(19) Europäisches Patentamt
European Patent Office
Office européen des brevets

(11) Veröffentlichungsnummer: **0 181 287**
**B1**

(12) # EUROPÄISCHE PATENTSCHRIFT

(45) Veröffentlichungstag der Patentschrifft: **21.03.90**

(51) Int. Cl.[5]: **A 61 K 31/565**, A 61 K 9/14, A 61 K 47/00

(21) Anmeldenummer: **85810500.0**

(22) Anmeldetag: **31.10.85**

(54) **Trockensubstanzen und stabile Suspensionen.**

(30) Priorität: **06.11.84 CH 5305/84**

(43) Veröffentlichungstag der Anmeldung:
**14.05.86 Patentblatt 86/20**

(45) Bekanntmachung des Hinweises auf die Patenterteilung:
**21.03.90 Patentblatt 90/12**

(84) Bennante Vertragsstaaten:
**AT BE CH DE FR GB IT LI LU NL SE**

(56) Entgegenhaltungen:
**EP-A-0 095 751**
**EP-A-0 117 811**
**DE-A-3 339 295**
**FR-A-2 068 447**
**US-A-4 391 755**

(73) Patentinhaber: **CIBA-GEIGY AG**
**Klybeckstrasse 141**
**CH-4002 Basel (CH)**

(72) Erfinder: **Geller, Leo, Dr.**
**Rudolf Wackernagel-Strasse 14**
**CH-4125 Riehen (CH)**
Erfinder: **Glanzmann, Peter**
**J.J. Balmerstrasse 7**
**CH-4053 Basel (CH)**

**EP 0 181 287 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die Erfindung betrifft Trockensubstanzen zur Herstellung von stabilen Suspensionen und stabile Suspensionen, die 4-Hydroxy- oder 4-$C_{2-12}$-Acyloxy-4-androsten-3,17-dione als Wirkstoffe enthalten und Verfahren zu deren Herstellung.

Wäßrige Suspensionen, insbesondere Injektionssuspensionen mit Steroidverbindungen, enthalten üblicherweise gemäß H. Sucker, P. Fuchs und P. Speiser, Pharmazeutische Technologie (1978), Verlag G. Thieme Stuttgart, Seite 616 als Suspensionshilfsmittel beispielsweise ein Netzmittel in niedrigen Konzentrationen, wie z. B. 0,1 bis 0,5 mg/ml Polysorbat oder 0,15 mg/ml Dioctylnatriumsulfosuccinat, ein Schutzkolloid, wie z. B. Natriumcarboxymethylcellulose oder Methylcellulose und einen Peptisator, wie z. B. Phosphatpuffer. Bekannterweise müssen diese Hilfsstoffe jedoch innerhalb sehr enger Grenzbereiche dosiert sein, damit die physikalische Stabilität der Suspension gewährleistet bleibt. Liegt die Zusammensetzung jedoch nicht innerhalb der engen Grenzbereiche optimal vor, so besteht die Gefahr, daß einerseits Kristallwachstum des suspendierten Wirkstoffes eintritt, oder daß der suspendierte Wirkstoff vorzeitig sedimentiert und sich nach einiger Zeit nicht mehr durch Aufschütteln homogen suspendieren läßt und gemäß den von in Remington, Pharmaceutical Sciences (1980), (A. Osol), S. 1457, aufgestellten idealen Anforderungen nicht mehr entspricht.

Es besteht ein echtes Bedürfnis, eine stabile wäßrige Suspension ohne die vorher aufgezählten Nachteile bereitzustellen. Darüber hinaus besteht ein Bedürfnis in der Medizin, Suspensionen als Tropfenformulierungen bereitzustellen, da in vielen Fällen Patienten leichter zur Einnahme von Tropfen zu bewegen sind als von Kapseln oder Tabletten. Insbesondere können injizierbare Suspensionen dem Patienten in jedem Zustand parenteral zugeführt werden.

Überraschenderweise konnten durch Lyophilisieren Trockensubstanzen zur Herstellung von stabilen Suspensionen und stabile Suspensionen an sich, insbesondere Injektionssuspensionen, die 4-Hydroxy- oder 4-$C_{2-12}$-Acyloxy-4-androsten-3,17-dione als Wirkstoff enthalten, erhalten werden, welche sich dadurch kennzeichnen, daß sie zusätzlich eine Kombination bestehend aus mindestens einem Phospholipid und mindestens einem festen Polyäthylenglykol in einem Verhältnis von 1 : 1 bis 1 : 10 aufweisen.

Es konnte überraschenderweise festgestellt werden, daß das Verhältnis der Kombination Phospholipid/Polyäthylenglykol für die durch Lyophilisieren erhältlichen Trockensubstanzen zur Herstellung von stabilen Suspensionen von großer Bedeutung ist.

Das Verhältnis der Kombination von Phospholipiden und Polyäthylenglykol liegt insbesondere im Bereich von 1 : 1 bis 1 : 5,0, vorzugsweise jedoch im Bereich von 1 : 1 bis 1 : 3,0.

Als Phospholipide für die erfindungsgemäße Verwendung als Netzmittel in Kombination mit Polyäthylenglykol kommen Gemische aus Phosphatidylcholin, Phosphatidyläthanolamin, N-Acylphosphatidyläthanolamin oder Phosphatidylinosit in Frage, insbesondere Phospholipide mit einem Phosphatidylcholin-Anteil von 30 - 98 %.

Es können z. B. folgende kommerziellen Produkte verwendet werden:

| Phosphatidylcholinghalt | Markenname |
|---|---|
| ca. 45 % | Epikuron 145® <br> Lipoid S45® |
| 80 - 85 % | Epikuron 170® <br> Lipoid E80® |
| 90 - 98 % | Epikuron 200® <br> Lipoid S100® |

Vor allem haben sich Kombinationen verschiedener Phospholipide als geeignet erwiesen.

Ein wesentlicher Aspekt der Erfindung liegt in der Auswahl des Polyäthylenglykols, da gefunden wurde, daß die erstrebte Stabilisierung der Suspension nur dann erzielt wird, wenn möglichst ein Polyäthylenglykol mit höherem Molekulargewicht verwendet wird. Besonders gut reproduzierbare Ergebnisse werden erhalten, wenn ausschließlich feste Polyäthylenglykole vom Molekulargewicht 1000 bis 6000 verwendet werden. Vorzugsweise werden für die Herstellung von Sus-pensionen, die insbesondere 4-Hydroxy-4-androsten-3,17-dion enthalten, Polyäthylenglykole vom Molekulargewicht 3000 - 4000 verwendet. Die Verwendung dieser festen Polyäthylenglykole hat eine leichte Viskositätserhöhung des Suspensionsmediums zur Folge, was die Suspendierbarkeit des Wirkstoffes verbessert und außerdem dienen diese Polyäthylenglykole als Gerüstbildner bei der nachfolgenden Lyophilisation (Gefriertrocknung). Feste Polyäthylenglykole des angegebenen Molekulargewichtes sind unter verschiedenen Bezeichnungen, beispielsweise unter Polyglykol oder Carbowax 1000®, 1500®, 3000®, 4000® oder 6000® im Handel.

Es war für den Fachmann nicht vorhersehbar, daß neben dem oben angegebenem Kombinationsbereich Phospholipid/Polyäthylenglykol das durchschnittliche Molekulargewicht einen so großen Einfluß auf die physikalische Stabilität der Suspension hat.

Wie bereits erwähnt, entsteht mit der Kombination der beiden Hilfsstoffe ein Suspensionsmedium mit leicht erhöhter Viskosität, in welchem der Wirkstoff in verschiedener Kristallstruktur und Konzentration homogen verteilbar ist. Ein besonderer Vorteil dieses Suspensionsmediums ist, daß sich erhaltene Suspensionen lyophilisieren lassen, wodurch sich eventuell noch vorhandene, d.h. sämtliche Stabilitätsprobleme, auf ein Minimum herabsetzen lassen. Das Lyophilisieren führt man z. B. aus, indem man eine bestimmte Menge der hergestellten Suspension üblicherweise in geeignete Behälter, wie Ampullen, z. B. Glasstechampullen (Vials) abfüllt und die abgefüllten Stechampullen anschließend bei ca. -40°C einfriert und danach bei einem Druck von 0,2 -

0,6 mbar und einer Endtemperatur von ca. 25° - 35°C lyophilisiert.

Die so entstandene Trockensubstanz wird vor der Verwendung als Suspension, beispielsweise vor der Injektion in physiologischen Lösungen, wie z. B. physiologischen Kochsalzlösungen oder auch physiologischen Zuckerlösungen, wie z. B. Glucoselösung oder im dest. Wasser rekonstituiert. Durch kurzes Schütteln entsteht wieder eine homogene Suspension, die aufgrund des enthaltenen Phospholipids nicht an der Behälterwand haftet und leicht und vollständig mit der Spritze entnehmbar ist.

Überraschenderweise gelingt es erstmalig durch Lyophilisieren, Trockensubstanzen und daraus rekonstituierbare Suspensionen, die 4-Hydroxy- oder 4-$C_{2-12}$-Acyloxy-4-androsten-3,17-dione enthalten, die physikalisch stabil und auch zur Injektion geeignet sind, herzustellen. Gegenstand der Erfindung sind demnach auch stabile Injektionssuspensionen, die als Injektionsformulierung zur besonderen Verwendung gelangen. Hiermit können stabile Suspensionen, insbesondere Injektionssuspensionen, die 4-Hydroxy- oder 4-$C_{2-12}$-Acyloxy-4-androsten-3,17-dione enthalten, vorzugsweise jedoch 4-Hydroxy-4-androsten-3,17-dione enthalten, als "ready for use" Präparate verwendet werden.

Die als orale oder injizierbare Formulierungen verwendbaren Suspensionen können je nach Bedarf mit den in der galenischen Pharmazie üblichen Zusätzen, Trägersubstanzen und/oder Geschmackskorrigentien und/oder Konservierungsmitteln und/oder Antioxidantien bei oraler oder parenteraler Gabe nach an sich bekannten Methoden verabreicht werden.

Für die Herstellung der Trockensubstanzen und den daraus rekonstituierbaren Suspensionen, geeignet für orale oder auch injizierbare Formulierungen können zwischen 10 und 1000 mg, vorzugsweise zwischen 50 und 250 mg, des Wirkstoffes der Formel I

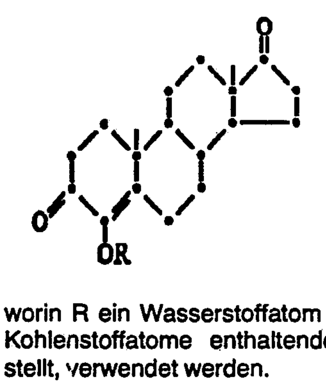

(I),

worin R ein Wasserstoffatom oder eine 2 bis 12 Kohlenstoffatome enthaltende Acylgruppe darstellt, verwendet werden.

Als Acylgruppen kommen die in der Steroidchemie üblicherweise verwendeten Acylgruppen in Frage, insbesondere die Acetyl-, Heptanoyl- oder Benzoylgruppe.

Vorzugsweise wird der Wirkstoff der Formel I in mikronisierter Form verwendet.

Wird der Wirkstoff in mikronisierter Form verwendet, so liegen die Partikel in einer Korngröße von 2 - 20 µm, vorzugsweise jedoch in einer mittleren Korngröße von 3 - 6 µm vor. Das Mikronisieren des Wirkstoffes geschieht mittels eines Ultraschalldesintegrators (z. B. Branson Sonifier) nach bekannten Verfahren (J. Pharm. Sci. 53 (9) 1040 - 45 (1965).

Bevorzugter Gegenstand der Erfindung ist demnach auch der Wirkstoff der Formel I in mikronisierter Form in einer Korngröße von 2 - 20 µm, vorzugsweise in einer mittleren Korngröße von 3 - 6 µm.

Die Verbindungen der allgemeinen Formel I sind bereits bekannt. Sie werden beschrieben als Aromatasehemmer, die die Umwandlung von 4-Androsten-3,17-dion zu Östrogenen an menschlichen Placentamikrosomen hemmen (Endocrinology 100 [1977] 1684 - 1695 und US-A-4 235 893). Die Verbindungen können die Entwicklung von östrogenabhängigen Brusttumoren bei Ratten unterdrücken.

Ferner wird die Verwendung der Verbindungen der Formel I in der DE-A-3 339 295 zur Prophylaxe und Therapie der Prostata Hyperplasie beschrieben.

Die folgenden Beispiele sollen die Erfindung verdeutlichen, jedoch nicht einschränken.

**Beispiel 1**

Zusammensetzung der Trockenstechampulle (Trockenvial):

| | |
|---|---|
| 4-Hydroxy-4-androsten-3,17-dion, mikronisiert | 250 mg |
| Epikuron 170® | 5 mg |
| Epikuron 200® | 45 mg |
| Carbowax 4000® | 150 mg |
| Thiomersal | 0,05 mg |

Die Herstellung erfolgt unter Stickstoff-Begasung und antimikrobiellen Bedingungen. In 6,407 g filtriertem und sterilisiertem Suspensionsmittel (17,0 mg Epikuron 170®, 152,5 mg Epikuron 200®, 508,5 mg Polyäthylenglykol 4000®) werden 847,5 mg steriles 4-Hydroxy-4-androsten-3,17-dion mikronisiert eingerührt und suspendiert. Zur Suspension werden 2,746 g sterilfiltrierte Thiomersal-Lösung (0,170 mg Thiomersal®) zugemischt. Der pH-Wert der Suspension wird durch Zugabe von ca. 1 Tropfen sterilfiltrierter 0,1n Natronlauge auf 5,0 - 6,0 gestellt. Durch Beschallen mit einem Ultraschall-Desintegrator wird die Suspension homogenisiert (desagglomeriert). Die Suspension wird zu 2,95 g in 6 ml Stechampullen abgefüllt. Die abgefüllte Suspension in den Stechampullen wird in einer Gefriertrocknungsapparatur bei -40°C eingefroren und danach bei einem Druck von 0,4 mbar und einer Endtemperatur von +35°C lyophilisiert.

Zusammensetzung des Rekonstituierungsmittels:

| | |
|---|---|
| Natriumchlorid | 18 mg |
| Wasser | bis zu 2 ml |

Zur Herstellung der gebrauchsfertigen Suspension werden mit einer Injektionsspritze zu einer Ampulle mit Trockensubstanz 2 ml Natriumchloridlösung 0,9 % gegeben. Nach kurzem Umschwenken entsteht wieder eine homogene Suspension, die 250 mg 4-Hydroxy-4-androsten-3,17-dion enthält.

**Beispiel 2**

Zusammensetzung der Trockenstechampulle (Trockenvial):

| | |
|---|---|
| 4-Hydroxy-4-androsten-3,17-dion mikronisiert, | 250,00 mg |
| Epikuron 200® | 50,00 mg |
| Carbowax 4000® | 75,00 mg |
| Thiomersal | 0,05 mg |
| Ascorbylpalmitat | 0,005 mg |

Unter Verwendung der entsprechend angegebenen Mengen erfolgt die Ausführung wie unter Beispiel 1 angegeben.

Zusammensetzung des Rekonstituierungsmittels:

| | |
|---|---|
| Wasser zur Injektion | 2 ml |

**Beispiel 3**

Zusammensetzung der Trockenstechampulle:

| | |
|---|---|
| 4-Hydroxy-4-androsten-3,17-dion mikronisiert, | 500 mg |
| Epikuron 170® | 50 mg |
| Polyglykol 3000® | 100 mg |
| Thiomersal | 0,05 mg |
| Tocopherol | 0,06 mg |

Unter Verwendung der entsprechend angegebenen Mengen erfolgt die Ausführung wie unter Beispiel 1 angegeben.

Zusammensetzung des Rekonstituierungsmittels:

| | |
|---|---|
| Natriumchlorid | 22,5 mg |
| Wasser zur Injektion | bis zu 2,5 ml |

**Beispiel 4**

Zusammensetzung der Trockenstechampulle (Trockenvial):

| | |
|---|---|
| 4-Hydroxy-4-androsten-3,17-dion, mikronisiert, | 1000 mg |
| Epikuron 170® | 50 mg |
| Epikuron 200® | 50 mg |
| Polyäthylenglykol | 300 mg |
| Thiomersal | 0,05 mg |
| Tocopherol | 0,12 mg |

Unter Verwendung der entsprechend angegebenen Mengen erfolgt die Ausführung wie unter Beispiel 1 angegeben.

Zusammensetzung des Rekonstituierungsmittels:

| | |
|---|---|
| Glucose | 250,0 mg |
| Wasser zur Injektion | bis zu 5 ml |

**Beispiel 5**

Herstellung von Mikrokristallen:

250 mg 4-Hydroxy-4-androsten-3,17-dion werden in 7,5 ml Aceton gelöst. Diese Lösung wird in einem Ultraschall-Desintegrator mit 75 ml Wasser vermischt. Die ausgefällten Mikrokristalle des Wirkstoffes 4-Hydroxy-4-androsten-3,17-dion werden auf einem Filter durch Filtration gesammelt und zweimal mit je 10 ml Wasser nachgewaschen. Die gewaschenen Mikrokristalle werden bei 40°C und einem Druck von > 100 mbar getrocknet.

**Patentansprüche**

1. Durch Lyophilisieren erhältliche Trockensubstanzen zur Herstellung von stabilen Suspensionen, die 4-Hydroxy- oder 4-$C_{2-12}$-Acyloxy-4-androsten-3,17-dione enthalten, dadurch gekennzeichnet, daß sie zusätzlich eine Kombination bestehend aus mindestens einem Phospholipid und mindestens einem festen Polyäthylenglykol im Verhältnis von 1 : 1 bis 1 : 10 enthalten.

2. Trockensubstanzen gemäß Anspruch 1, dadurch gekennzeichnet, daß sie die Kombination im Verhältnis von 1 : 1 bis 1 : 5 aufweist.

3. Trockensubstanzen gemäß Ansprüchen 1 und 2, dadurch gekennzeichnet, daß sie die Kombination im Verhältnis von 1 : 1 bis 1 : 3 aufweist.

4. Trockensubstanzen gemäß Ansprüchen 1 - 3, dadurch gekennzeichnet daß als Polyäthylenglykol ein festes Polyäthylenglykol von einem Molekulargewicht 1000 bis 6000 vorliegt.

5. Trockensubstanzen gemäß Ansprüchen 1 - 4, die 4-Hydroxy-4-androsten-3,17-dion enthalten, dadurch gekennzeichnet, daß als Polyäthylenglykol ein festes Polyäthylenglykol von einem Molekulargewicht 3000 bis 4000 vorliegt.

6. Verfahren zur Herstellung von durch Lyophilisieren erhältlichen Trockensubstanzen, geeignet zur Herstellung von stabilen Suspensionen, die 4-Hydroxy-oder 4-$C_{2-12}$-Acyloxy-4-androsten-3,17-dione enthalten, dadurch gekennzeichnet, daß man 4-Hydroxy- oder 4-Acyloxy-4-androsten-3,17-dione in Gegenwart einer festen Kombination bestehend aus mindestens einem Phospholipid und mindestens einem festen Polyäthylenglykol in einem Verhältnis von 1 : 1 bis 1 : 10 als Netzmittel und als Mittel zur Erhöhung der Viskosität des Suspensionsmittels in einem wäßrigen Suspensionsmittel suspendiert und die erhaltenen Suspensionen lyophilisiert.

7. Verfahren zur Herstellung von Trockensubstanzen, die 4-Hydroxy- oder 4-Acyloxy-4-androsten-3,17-dione enthalten gemäß Anspruch 6, dadurch gekennzeichnet, daß man eine Kombination bestehend aus mindestens einem Phospholipid und mindestens einem festen Polyäthylenglykol in einem Verhältnis von 1 : 1 bis 1 : 5 als Netzmittel und als Mittel zur Erhöhung der Viskosität des Suspensionsmittels verwendet.

8. Verfahren zur Herstellung von Trockensubstanzen gemäß Ansprüchen 6 und 7, dadurch gekennzeichnet, daß man eine Kombination bestehend aus mindestens einem Phospholipid und mindestens einem festen Polyäthylenglykol in einem Verhältnis von 1 : 1 bis 1 : 3 als Netzmittel und als Mittel zur Erhöhung der Viskosität des Suspensionsmittels verwendet.

9. Verfahren zur Herstellung von Trockensubstanzen gemäß Ansprüchen 6 - 8, dadurch gekennzeichnet, daß man als Polyäthylenglyol ein festes Polyäthylenglykol von einem Molekulargewicht 1000 bis 6000 verwendet.

10. Verfahren zur Herstellung von Trockensubstanzen gemäß Ansprüchen 6 - 9, die 4-Hydroxy-4-androsten-3,17-dion enthalten, dadurch gekennzeichnet, daß man als Polyäthylenglykol ein festes Polyäthylenglykol von einem Molekulargewicht 3000 bis 4000 verwendet.

11. Verfahren zur Herstellung von Trockensubstanzen gemäß Ansprüchen 6 - 10, dadurch gekennzeichnet, daß man 4-Hydroxy- oder 4-$C_{2-12}$-Acyloxy-4-androsten-3,17-dione im mikronisierten Zustand verwendet.

12. Verfahren nach Anspruch 11, dadurch gekennzeichnet, daß man 4-Hydroxy- oder 4-$C_{2-12}$-Acyloxy-4-androsten-3,17-dione in mikronisierter Form in einer Korngröße von 2 - 20 µm verwendet.

13. Verfahren nach Anspruch 11, dadurch gekennzeichnet, daß man 4-Hydroxy- oder 4-$C_{2-12}$-Acyloxy-4-androsten-3,17-dione in mikronisierter Form in einer Korngröße von 3 - 6 µm verwendet.

14. Stabile Suspensionen enthaltend eine Trokkensubstanz in der Zusammensetzung gemäß einem der Ansprüche 1 - 5 in einem wäßrigen Suspensionsmittel.

15. Stabile Suspensionen gemäß Anspruch 14, dadurch gekennzeichnet, daß sie in für die orale oder parenterale Verabreichung geeigneter Form vorliegen.



**Claims**

1. Dry substances obtainable by lyophilisation for the preparation of stable suspensions and containing 4-hydroxy- or 4-$C_{2-12}$-acyloxy-4-androstene-3,17-diones, which substances additionally contain a combination consisting of at least one phospholipid and at least one solid polyethylene glycol in a ratio of 1 : 1 to 1 : 10.

2. Dry substances according to claim 1, which contain the combination in a ratio of 1 : 1 to 1 : 5.

3. Dry substances according to claims 1 and 2, which contain the combination in a ratio of 1 : 1 to 1 : 3.

4. Dry substances according to claims 1 - 3, wherein the polyethylene glycol is a solid polyethylene glycol having a molecular weight of 1000 to 6000.

5. Dry substances according to claims 1 - 4, which contain 4-hydroxy-4-androstene-3,17-dione, wherein the polyethylene glycol is a solid polyethylene glycol having a molecular weight of 3000 to 4000.

6. A process for the preparation of dry substances obtainable by lyophilisation and suitable for the preparation of stable suspensions, said substances containing 4-hydroxy- or 4-$C_{2-12}$-acyloxy-4-androstene-3,17-diones, which process comprises suspending 4-hydroxy- or 4-acyloxy-4-androstene-3,17-diones in an aqueous suspension vehicle, in the presence of a combination consisting of at least one phospholipid and at least one solid polyethylene glycol in a ratio of 1 : 1 to 1 : 10 as wetting agent and as agent for increasing the viscosity of said suspension vehicle, and lyophilising the resultant suspensions.

7. A process according to claim 6 for the preparation of dry substances which contain 4-hydroxy- or 4-acyloxy-4-androstene-3,17-diones, wherein a combination consisting of at least one phospholipid and at least one solid polyethylene glycol in a ratio of 1 : 1 to 1 : 5 is used as wetting agent and as agent for increasing the viscosity of the suspension vehicle.

8. A process for the preparation of dry substances according to claims 6 and 7, wherein a combination consisting of at least one phospholipid and at least one solid polyethylene glycol in a ratio of 1 : 1 to 1 : 3 is used as wetting agent and as agent for increasing the viscosity of the suspension vehicle.

9. A process for the preparation of dry substances according to claims 6 - 8, wherein a solid polyethylene glycol having a molecular weight of 1000 to 6000 is used as polyethylene glycol.

10. A process for the preparation of dry substances according to claims 6 - 9 which contain 4-hydroxy-4-androstene-3,17-dione, wherein a solid polyethylene glycol having a molecular weight of 3000 to 4000 is used as polyethylene glycol.

11. A process for the preparation of dry substances according to claims 6 - 10, wherein micronised 4-hydroxyor 4-$C_{2-12}$-acyloxy-4-androstene-3,17-diones are used.

12. A process according to claim 11, wherein micronised 4-hydroxy- or 4-$C_{2-12}$-acyloxy-4-androstene-3,17-diones having a particle size of 2-20 µm are used.

13. A process according to claim 11, wherein micronised 4-hydroxy- or 4-$C_{2-12}$-acyloxy-4-androstene-3,17-diones having a particle size of 3-6 µm are used.

14. Stable suspensions containing a dry substance of the composition as claimed in any one of claims 1 - 5, in an aqueous suspension vehicle.

15. Stable suspensions according to claim 14 in a form suitable for oral or parenteral administration.

**Revendications**

1. Matières sèches, obtenues par lyophilisation, pour la préparation de suspensions stables, contenant des 4-hydroxy- ou 4-(acyloxy en C 2 - C 12)-4-androstène3,17-diones, caractérisées en ce qu'elles contiennent en outre une combinaison consistant en au moins un phospholipide et au moins un polyéthylène-glycol solide dans des proportions relatives de 1 : 1 à 1 : 10.

2. Matières sèches selon la revendication 1, caractérisées en ce qu'elles contiennent la combinaison à des proportions relatives de 1 : 1 à 1 : 5.

3. Matières sèches selon les revendications 1 et 2, caractérisées en ce qu'elles contiennent la combinaison à des proportions relatives de 1 : 1 à 1 : 3.

4. Matières sèches selon les revendications 1 à 3, caractérisées en ce que le polyéthylène-glycol est un polyéthylène-glycol solide de poids moléculaire 1000 à 6000.

5. Matières sèches selon les revendications 1 à 4, contenant de la 4-hydroxy-4-androstène-3,17-dione, caractérisées en ce qu'elles contiennent en tant que polyéthylène-glycol un polyéthylène-glycol solide de poids moléculaire 3000 à 4000.

6. Procédé de préparation de matières sèches obtenues par lyophilisation et convenant à la préparation de suspensions stables contenant de la 4-hydroxy- ou des 4-(acyloxy en C 2 - C 12)-4-androstène-3,17-diones, caractérisé en ce que l'on met la 4-hydroxy- ou les 4-(acyloxy-4-androstène-3,17-diones en suspension dans un milieu de suspension aqueux en présence d'une combinaison consistant en au moins un phospholipide et au moins un polyéthylène-glycol solide, dans des proportions relatives de 1 : 1 à 1 : 10, faisant fonction d'agent mouillant et d'additif accroissant la viscosité du milieu de suspension, et on lyophilise la suspension obtenue.

7. Procédé de préparation de matières sèches contenant de la 4-hydroxy- ou des 4-acyloxy-4-androstène-3,17-diones selon la revendication 6, caractérisé en ce que l'on utilise une combinaison consistant en au moins un phospholipide et au moins un polyéthylène-glycol solide dans des proportions relatives de 1 : 1 à 1 : 5 en tant qu'agent mouillant et additif accroissant la viscosité du milieu de suspension.

8. Procédé de préparation de matières sèches selon les revendications 6 et 7, caractérisé en ce que l'on utilise une combinaison consistant en au moins un phospholipide et au moins un polyéthylène-glycol solide dans des proportions relatives de 1 : 1 à 1 : 3 en tant qu'agent mouillant et additif accroissant la viscosité du milieu de suspension.

9. Procédé de préparation de matières sèches selon les revendications 6 à 8, caractérisé en ce que le polyéthylène-glycol utilisé est un polyéthylène-glycol solide de poids moléculaire 1000 à 6000.

10. Procédé de préparation de matières sèches selon les revendications 6 à 9, contenant de la 4-hydroxy-4-androstène-3,17-dione, caractérisé en ce que l'on utilise en tant que polyéthylène-glycol un polyéthylène-glycol solide de poids moléculaire 3000 à 4000.

11. Procédé de préparation de matières sèches selon les revendications 6 à 10, caractérisé en ce que l'on utilise de la 4-hydroxy- ou des 4-(acyloxy en C 2 - C 12)-4-androxtène-3,17-diones à l'état micronisé.

12. Procédé selon la revendication 11, caractérisé en ce que l'on utilise de la 4-hydroxy- ou des 4-(acyloxy en C 2-C 12)-4-androstène-3,17-diones à l'état micronisé à une dimension de grain de 2 à 20 µm.

13. Procédé selon la revendication 11, caractérisé en ce que l'on utilise de la 4-hydroxy- ou des 4-(acyloxy en C 2-C 12)-4-androstène-3,17-diones à l'état micronisé à une dimension de grain de 3 à 6 µm.

14. Suspensions stables contenant des matières sèches à la composition indiquée selon l'une des

revendications 1 à 5, dans un milieu de suspension aqueux.

15. Suspensions stables selon la revendication 14, caractérisées en ce qu'elles sont sous une forme appropriée à l'administration orale ou parentérale.